# FASCICULE DE BREVET EUROPEEN

(11) **EP 1 175 221 B1**
(45) Date de publication et mention de la délivrance du brevet: **23.05.2007**
(21) Numéro de dépôt: 00925413.7
(22) Date de dépôt: 05.05.2000
(51) Int. Cl.: A61K 35/74, A61K 35/20, C12N 1/20, A23C 9/123, A61P 25/22, C12R 1/46, C12R 1/225, C12R 1/23

(54) **BACTERIES LACTIQUES A PROPRIETES ANXIOLYTIQUES, ET LEURS UTILISATIONS**
MILCHSÄUREBAKTERIEN MIT ANXIOLYTISCHER WIRKUNG, UND IHRE VERWENDUNGEN
LACTIC ACID BACTERIA WITH ANXIOLYTIC PROPERTIES AND USES THEREOF

(30) Priorité: 06.05.1999 FR 9905760
(43) Date de publication de la demande: 30.01.2002
(73) Titulaire: COMPAGNIE GERVAIS DANONE, 92302 Levallois-Perret (FR)
(72) Inventeur: ANTOINE, Jean-Michel, F-94700 Maisons-Alfort (FR); CAYUELA, Chantal, F-75015 Paris (FR); DEGIVRY, Marie-Christine, F-92350 Le Plessis Robinson (FR); LATGE, Christian, F-91430 Igny (FR); POSTAIRE, Eric, F-92170 Vanves (FR)
(74) Mandataire: Vialle-Presles, Marie José
(86) Numéro de dépôt international: PCT/FR2000/001238
(87) Numéro de publication internationale: WO 2000/067696

(56) Documents cités:
- EP-A- 0 652 285
- WO-A-97/45530
- WO-A-98/05343
- PATENT ABSTRACTS OF JAPAN vol. 1999, no. 09, 30 juillet 1999 (1999-07-30) & JP 11 100328 A (THE CALPIS CO LTD), 13 avril 1999 (1999-04-13) -& EP 1 018 341 A 12 juillet 2000 (2000-07-12)
- PATENT ABSTRACTS OF JAPAN vol. 1999, no. 09, 30 juillet 1999 (1999-07-30) & JP 11 098978 A (THE CALPIS CO LTD), 13 avril 1999 (1999-04-13) -& EP 1 016 709 A 5 juillet 2000 (2000-07-05)

## Description

L'invention est relative à de nouvelles souches de ferments lactiques et à leur utilisation pour la production d'aliments dotés de propriétés anxiolytiques.

Le stress, qui est une réaction de défense de l'organisme vis à vis d'agressions extérieures, engendre notamment au niveau du système nerveux, des perturbations biologiques qui peuvent évoluer en pathologies diverses. Celles ci peuvent se manifester directement au niveau psychologique, notamment sous forme d'anxiété, et/ou prendre la forme de manifestations somatiques, telles qu'hypertension artérielle, ulcérations gastriques, etc. Elles évoluent fréquemment vers des états chroniques.

Pour traiter les conséquences du stress, qu'il s'agisse de l'anxiété ou des manifestations somatiques, on utilise fréquemment des anxiolytiques, et notamment des benzodiazépines. Cependant ces médicaments qui peuvent engendrer rapidement un phénomène d'accoutumance, peuvent ne pas convenir à un traitement au long cours. D'autre part, leur action anxiolytique s'accompagne notamment de propriétés sédatives qui peuvent entraîner des effets secondaires gênants.

Certaines recherches ont mis en évidence l'existence dans le lait, ou dans des produits dérivés de celui-ci, de substances dotées de propriétés pharmacologiques vis à vis des pathologies liées au stress. Notamment, la Demande PCT WO 98/05343, aux noms de CALPIS CO. et GROUPE DANONE, décrit un agent possédant des propriétés anti-stress se traduisant par un effet anti-hypertenseur. Cet agent est obtenu par fermentation du lait avec une bactérie lactique du genre *Lactobacillus,* notamment *Lactobacillus helveticus.* Il résiste à la stérilisation, et peut être utilisé pour préparer des aliments participant à la prévention et/ou au traitement des effets périphériques (somatiques) du stress, et pouvant être consommés dans le cadre de l'alimentation habituelle, de manière régulière et sur de longues périodes. Aucun effet central, notamment anxiolytique résultant de l'administration de cet agent n'est mis en évidence dans la Demande PCT WO 98/05343.

La Demande EP 95402697 au nom de : SOCIETE COOPERATIVE LAITIERE AGRICOLE D'ARTOIS ET DES FLANDRES, LA PROSPERITE FERMIERE, décrit un décapeptide résultant de l'hydrolyse trypsique de la caséine αₛ1. L'administration par voie parentérale de ce décapeptide, ou de l'hydrolysat trypsique le comprenant induit un effet anxiolytique et anticonvulsivant du même type que celui des benzodiazépines. En revanche, ce document ne fournit aucune indication concernant l'activité de cet hydrolysat par voie orale, et la possibilité de l'incorporer dans des aliments afin de leur conférer des propriétés anti-stress.

Les Inventeurs ont maintenant mis en évidence, dans des produits laitiers fermentés par certaines souches de bactéries lactiques, l'existence d'un ou plusieurs principe (s) actif (s) capables d'induire lorsqu'ils sont administrés par voie orale un effet anti-stress de type anxiolytique, sans posséder les propriétés sédatives des benzodiazépines.

La présente invention a pour objet une souche de bactérie lactique choisie parmi le groupe constitué par :
- la souche de *Streptococcus thermophilus* S242 déposée le 24 février 1999 selon le traité de Budapest auprès de la CNCM (Collection Nationale de Cultures de Micro-organismes), 28 rue du Docteur Roux, 75724 Paris, sous le numéro I-2130 ;
- la souche de *Streptococcus thermophilus* S003 déposée le 24 février 1999 selon le traité de Budapest auprès de la CNCM (Collection Nationale de Cultures de Micro-organismes), 28 rue du Docteur Roux, 75724 Paris, sous le numéro I-2129 ;
- la souche de *Lactobacillus gasseri* L012 déposée le 24 février 1999 selon le traité de Budapest auprès de la CNCM (Collection Nationale de Cultures de Micro-organismes), 28 rue du Docteur Roux, 75724 Paris, sous le numéro I-2131 ;
- la souche de *Lactobacillus acidophilus* L030 déposée le 24 février 1999 selon le traité de Budapest auprès de la CNCM (Collection Nationale de Cultures de Micro-organismes), 28 rue du Docteur Roux, 75724 Paris, sous le numéro 1-2132.

La présente invention a également pour objet l'utilisation d'au moins une des souches de bactéries lactiques nouvellement isolées et caractérisées mentionnées ci-dessus, pour l'obtention d'un anxiolytique non-sédatif.

Les caractéristiques des souches de bactéries lactiques conformes à l'invention sont résumées dans les Tableaux I et II ci-dessous.

**TABLEAU I**

| Souche | S003 | S242 | L012 | L030 |
|---|---|---|---|---|
| N°CNCM | I-2129 | I-2130 | I-2131 | I-2132 |
| Morphologie | Assez gros coques en majorité en longues chaînes et quelques moyennes et courtes chaînes | Petits coques par 2 ou en courtes chaînes | Petits bacilles fins peu colorés souvent cocobacillaires | Bacilles de taille moyenne isolés ou en petites chaînes |
| | Gram + | Gram + | Gram + | Gram + |
| Métabolisme | Oxydase : - | Oxydase : - | Oxydase:- | Oxydase:- |
| | Catalase : - | Catalase : - | Catalase : - | Catalase : - |
| | Aéro-anaérobie | Aéro-anaérobie | Nitrate réductase : - | Nitrate réductase: - |
| | facultatif | facultatif | Nitrite réductase : - | Nitrile réductase : - |
| | | | Aéro-anaérobie | Aéro-anaérobie |
| T° de croissance | 37-44 °C | 37-44 °C | 37-44 °C | 37-44 °C |

**TABLEAU II**

| Souche | S003 | S242 | L012 | L030 |
|---|---|---|---|---|
| N° CNCM | I-2129 | I-2130 | I-2131 | I-2132 |
| Galactose | - | - | + | + |
| D-Glucose | + | + | + | + |
| D-Fucose | - | - | + | + |
| D-Mannose | - | - | + | + |
| N-Acetyl-Glucosamine | - | - | + | + |
| Amygdaline | - | - | + | + |
| Arbutine | - | - | +/- | + |
| Esculine | - | - | + | + |
| Salicine | - | - | + | + |
| Cellobiose | - | - | + | + |
| Matose | - | - | + | + |
| Saccharose | + | + | + | + |
| Threalose | - | - | + | + |
| β-Gentobiose | - | - | + | + |
| D-Tagatose | - | - | + | - |
| D-Turanose | - | - | - | + |
| Lactose | + | + | + | + |
| D-Raffinose | - | - | - | + |
| Melibiose | - | - | - | + |
| α-Methyl-D-Glucoside | - | - | - | + |

La présente invention concerne en particulier l'utilisation, pour l'obtention d'un anxiolytique non-sédatif, d'au moins une souche de *Streptococcus thermophilus* choisie parmi la souche S242 et la souche S003, associée avec la souche de *S*. *thermophilus* S147 déposée auprès de la CNCM le 30 décembre 1994, sous le numéro I-1520.

La présente invention concerne également des ferments lactiques comprenant au moins une souche conforme à l'invention, avantageusement associée avec une autre souche de bactérie lactique.

Selon un mode de réalisation préféré d'un ferment lactique conforme à l'invention, il comprend au moins la souche de *S. thermophilus* S242 et/ou la souche de *S. thermophilus* S003, avantageusement assossiée(s) avec la souche de *S. thermophilus* S147.

Pour l'obtention d'un anxiolytique non sédatif, on effectue la mise en culture d'au moins une souche de bactérie lactique conforme à l'invention, notamment la mise en culture d'un ferment lactique comprenant au moins la souche de *S. thermophilus* S242 et/ou la souche de *S. thermophilus* S003 avantageusement associée(s) avec la souche de S. *thermophilus* S147, et éventuellement avec la souche de *L. gasseri* L012 et/ou la souche de *L. acidophilus* L030, dans un milieu approprié, c'est à dire du lait ou un milieu à base de lait. Il peut notamment être choisi parmi les laits de différentes espèces de mammifères, éventuellement partiellement ou totalement écrémés, les produits résultant de la dilution ou de la concentration de ces laits, tels que par exemple des rétentats d'ultrafiltration ou de diafiltration, les milieux à base de lait, tels que par exemple les bases pour aliments lactés, les bases pour yoghourt, etc... Ces laits peuvent en outre être supplémentés avec par exemple, du lactose, des minéraux, des vitamines, des matières grasses ou non, des matières sèches hydrophiles du lait, des protéines de soja, des extraits végétaux, etc.

Ce milieu est ensemencé avec une culture comprenant au moins une souche de bactérie lactique conforme à l'invention, comprenant de préférence 10⁷ à 10⁹ bactéries/ml. Les conditions optimales de culture varient selon la souche ou le mélange de souches concernés. Par exemple, si la culture d'ensemencement est constituée par une souche ou un mélange de souches de *Streptococcus thermophilus,* la culture sera effectuée pendant environ 24 h et la température optimale de culture sera comprise entre 30 et 44°C ; si la culture d'ensemencement est constituée par une souche ou un mélange de souches de *L. acidophilus* ou de *L. gasseri,* la culture sera effectuée pendant environ 16 h et la température optimale de culture sera comprise entre 37 et 44°C. Dans le cas où la culture d'ensemencement associe au moins une souche de *Streptococcus thermophilus* avec au moins une souche de *L. acidophilus* ou de *L. gasseri,* la culture sera effectuée pendant 16 à 24 h, et la température optimale de culture sera comprise entre 37 et 44°C.

Les anxiolytiques non-sédatifs obtenus conformément à l'invention sont actifs par voie orale. Leur prise régulière entraîne au bout de quelques jours une diminution de l'anxiété, sans baisse de l'activité ; elle peut être prolongée pendant une très longue durée, sans effet d'accoutumance, et sans apparition d'effets secondaires défavorables. Ces propriétés, ainsi que le fait qu'il soit possible de les obtenir à partir de microorganismes appartenant à des espèces couramment utilisées dans l'industrie alimentaires, et de matières premières également à usage alimentaire, permettent de les utiliser dans le cadre la fabrication d'aliments ou de compléments alimentaires, dont la consommation exerce un effet bénéfique dans le cadre du traitement ou de la prévention des états pathologiques liés au stress.

La présente invention a également pour objet les produits ou compléments alimentaires comprenant un anxiolytique non-sédatif obtenu conformément à l'invention.

Ces produits alimentaires peuvent être en particulier des produits comprenant au moins une souche de bactérie lactique ou un ferment lactique conforme à l'invention, et constitués par du lait ou un milieu à base de lait, fermenté par ladite souche ou ledit ferment. Des produits alimentaires conformes à l'invention peuvent également être préparés par addition à tout aliment fermenté ou non, d'une préparation comprenant un anxiolytique non-sédatif obtenu conformément à l'invention.

Les effets bénéfiques des aliments conformes à l'invention apparaissent généralement à partir d'une dose de 5ml/jour/kg de poids, dans le cas d'un lait fermenté contenant une population totale (pouvant être constituée par une ou plusieurs souches de bactéries conformes à l'invention) de bactéries lactiques conformes à l'invention d'au moins 10⁵ UFC/ml. Des doses très supérieures peuvent toutefois être absorbées, sans effets secondaires néfastes.

La présente invention sera mieux comprise à l'aide du complément de description qui va suivre, qui se réfère à des exemples non-limitatifs, illustrant les propriétés anxiolytiques de produits laitiers fermentés obtenus conformément à l'invention.

### EXEMPLE 1 : PREPARATION DES DIFFERENTS PRODUITS LAITIERS TESTES.

Les souches et ferments utilisés dans les différentes expérimentations qui vont suivre sont les suivantes :
- souche *S. thermophilus* S242
- souche *S. thermophilus* S147
- souche *S. thermophilus* S003
- Ferment F042 : (ferment associant les 3 souches S242, S147, S003 de *S. thermophilus)*
- souche *L. gasseri* L012
- souche *L. acidophilus* L030
Fabrication des produits :
Du lait UHT à 0% de matière grasse a été traité à 95°C pendant 10 minutes, puis refroidi à 57 ou 44°C en fonction de la température des fermentations avant d'être ensemencé avec des cultures actives des souches utilisées pour obtenir la préparation souhaitée.

L'ensemencement avec les souches de *S. thermophilus* S242, S147, et S003 utilisées individuellement, est effectué à raison de 1% (v/v) d'une culture à 10⁸ à 10⁹ UFC/ml sur le lait stérile additionné d'autolysat de levure (3 h à 44°C).

L'ensemencement avec le ferment F042 est effectué à raison de 30 g/100 1 d'un mélange entre 10⁸ et 10⁹ UFC/ml concentré congelé des souches S242, S147 et 5003. L'incubation est réalisée à 44°C ; lorsque le pH atteint environ 4,5 la préparation est transférée à 4°C, et conservée à cette température jusqu'à utilisation.

Les populations bactériennes en fin de fermentation dans les produits fermentés par les souches séparées sont : *Streptococcus thermophilus* S242 : 1,2.10⁹ UFC/ml ; *Streptococcus thermophilus* S147 : 1,8.10⁹ UFC/ml ; *Streptococcus thermophilus* S003 : 1,3.10⁷ UFC/ml.

Dans le produit fermenté par le mélange F042, la concentration en *Streptococcus thermophilus* (S242+S147+S003) est de 1,9.10⁸ UFC/ml.

L'ensemencement avec les souches de *L. acidophilus* L012 et L030 est effectué à raison de 3% (v/v) d'une culture à 10⁸ à 10⁹ UFC/ml en milieu MRS neutre (16 h à 37°C). L'incubation est effectuée à 37°C ; lorsque le pH atteint une valeur comprise entre 4,5 et 5,9, la préparation est transférée à 4°C, et conservée à cette température jusqu'à utilisation.

Le sérum filtré du lait fermenté par le ferment F042 est préparé en centrifugeant 500 ml de lait fermenté à 10000 tr/min (centrifugeuse SORVAL RC5B, rotor GSA) 15 minutes 2 fois puis en filtrant le surnageant sur filtre NALGÈNE en nitrate de cellulose (HIGH-BINDING PROTEIN) 0,45 µm puis 0,22 µm).

### EXEMPLE 2 : EVALUATION DE L'EFFET ANXIOLYTIQUE D'UN MELANGE DE 3 STREPTOCOQUES THERMOPHILES

Les produits testés sont les suivants :
3S : Lait fermenté par le ferment F042 (comportant 3 *S. thermophilus* S242, S147, S003)
3S+P : Lait fermenté par le ferment F042 + Passiflore
3ST : Lait fermenté par le ferment F042 puis thermisé à 73°C pendant 30 secondes avec homogénéisation à 100 bars.
L : Lait témoin stérilisé à 130°C pendant 30 secondes

L'effet anxiolytique est évalué en utilisant le modèle d'Enfouissement Défensif Conditionné, qui a été mis au point à partir de la procédure de J.P. PINEL et D. TREIT (PINEL et TREIT , J. Comp. & Physiol. Psychol., 92, 708-712, 1978 ; TREIT et al., Pharmacol. Biochem. Behav., 15, 619-626, 1981). Ce modèle utilise la propension naturelle de la plupart des rongeurs à enfouir une source de stimulation stressante (TREIT, Pharmacol. Biochem. Behav., 22, 1, 47-52, 1985 ; MEERT et COLPAERT, Psychopharmacology, 88, 445-450, 1986 ; CRAFT et al., Pharmacol. Biochem. Behav., 30, 3, 775-780, 1988 ; TREIT, Pharmacol. Biochem. Behav., 36, 203-205, 1990).

### Animaux

Soixante douze rats mâles Wistar/AF EOPS (Centre d'élevage IFFA-CREDO, 69 - St-Germain sur l'Arbresle, France) d'un poids de 320 à 340 g ont été utilisés. A la réception les rats ont été pesés, marqués et répartis par groupes de 4 dans des cages en polycarbonate de type F (48 x 27 x 20 cm, U.A.R., 91 - Epinay-Sur-Orge, France). Les animaux ont été stabulés dans une animalerie climatisée, à une température de 22-24°C. Les rats ont disposé de nourriture (croquettes M25, Ets PIÉTREMENT, 77 - Provins, France) et de boisson ad *libitum.* Ils ont été soumis à un cycle lumière-obscurité de 12 heures (lumière de 24 h à 12 h).

Après une familiarisation d'une semaine aux conditions du laboratoire, les 72 rats ont été répartis au hasard en 6 groupes (n = 12). Les rats des différents groupes ont tous été manipulés de la même façon et dans les mêmes conditions.

### Matériels

### Cage d'expérimentation

L'habituation et les tests ont été réalisés dans une cage transparente de dimensions 44 x 28 x 18 cm, dont le fond est tapissé de sciure sur une épaisseur de 5 cm. Au milieu d'une face latérale, un orifice permet la fixation d'une sonde électrique, 2 cm au-dessus du niveau de la sciure.

### Sonde électrique

Il s'agit d'une sonde en plexiglas de dimensions 7 x 2 x 0,5 cm, recouverte d'un circuit imprimé en cuivre dont l'écartement des fils est de 1 mn, choisi afin qu'une patte de rat se posant sur la sonde fasse aisément contact entre eux, permettant ainsi le passage du courant.

### Appareillage électrique

La sonde est reliée à un générateur de chocs électriques de 0 à 8 mA à déclenchement manuel (OPEN-SYSTEMS, 54 - Maxéville, France).

### Enregistrement et dépouillement des séquences comportementales

Dans la pièce d'expérimentation faiblement éclairée, une caméra vidéo a été placée devant la cage du dispositif, à une distance de un mètre. La caméra est reliée à un magnétoscope et à un moniteur de contrôle, placés dans une pièce adjacente avec le générateur de chocs électriques.

### Procédure expérimentale

L'expérimentation a été réalisée en double insu.

### Habituation

Pendant les 2 jours précédant le premier test, les rats ont été transportés de l'animalerie jusque dans la pièce d'expérimentation et introduits dans le dispositif expérimental afin de les habituer à la fois aux manipulations et à la cage du dispositif en l'absence de l'électrode. Chaque groupe de 4 rats a été placé dans le dispositif pendant 20 minutes. La sciure est changée, égalisée, à une hauteur uniforme de 5 cm, avant la période d'habituation de chacun des groupes.

### Expérience

Le test d'Enfouissement Défensif Conditionné est réalisé dans les 5 premières heures de la phase d'obscurité, phase où les rats sont les plus actifs.

La sonde électrique est insérée dans la cage avant le début du premier test. Chaque rat est déposé dans la cage d'expérimentation du côté opposé à la sonde.

Lors du premier test (test 1), un choc électrique unique, de faible intensité (2 mA), est délivré à l'animal au moment où il pose une patte antérieure pour la première fois sur la sonde.

Suite au choc électrique, le comportement de chaque rat est enregistré pendant 5 minutes. Lors des deux tests suivants (tests 2 et 3), le rat est remis dans la cage d'expérimentation en présence de la sonde inactive. La sonde est nettoyée après chaque rat et la sciure est changée et égalisée à une hauteur uniforme de 5 cm avant la session de test de chaque cage de rats.

### Administration des produits

Les produits L, 3ST, 3S et 3S+P ont été administrés par voie orale quotidiennement aux mêmes heures, aux doses de 5 ml/kg au moyen de seringues munies de sondes de gavage intragastriques (HARVARD APPARATUS, 91 - Les Ulis, France). Les tests d'enfouissement (test 1 au jour 6 et test 2 au jour 8) ont été réalisés 6 heures après l'administration des produits).

Le témoin positif est le diazépam (3 mg/kg), a été mis en suspension dans une solution aqueuse de méthylcellulose à 1% et a été administré quotidiennement, par voie orale, pendant la même période. Les jours de test, il a été administré 1 heure avant le test d'enfouissement.

Les quatre rats de chaque cage ont reçu chacun un produit différent.

### Variables

- durée d'enfouissement de la sonde : les séquences d'enfouissement sont remarquablement stéréotypées après le choc ; le rat fait face à la sonde électrique et projette de la sciure dans sa direction par un rapide mouvement alternatif des pattes antérieures ;
- nombre d'étirements vis-à-vis de la sonde : le rat s'immobilise à quelques centimètres de la sonde et allonge son cou pour approcher le museau de la sonde ;
- nombre d'approches de la sonde : toute approche de la tête du rat en direction de la sonde à une distance de moins de 3 cm ;
- nombre de fuites vis-à-vis de la sonde : toute fuite précipitée du rat suite à une approche ou à un contact avec la sonde ;
- pourcentage d'approches de la sonde suivies de fuites = (nombre de fuites/nombre d'approches) x 100.

### Analyse statistique

Les données ont été classées par ordre croissant pour les variables suivantes : durée d'enfouissement, nombre d'étirements et pourcentage d'approches suivies de fuites. Au sein des diverses variables, un rang a été attribué à chaque donnée et la somme des rangs constitue le score global de stress de chaque rat.

Le test de KRUSKAL-WALLIS a été utilisé pour mettre en évidence l'existence d'une hétérogénéité parmi les groupes. Le test de MANN-WHITNEY a servi pour comparer les groupes DZP, 3ST, 3S et 3S+P au produit témoin L. Les résultats sont exprimés en médiane et écart interquartile (Quartile Inférieur - Quartile Supérieur).

Les traitements statistiques et graphiques ont été réalisés à l'aide des logiciels STATVIEW 4.1 et DELTAGRAPH PRO 3.5.

### Résultats

### Score global de stress au cours du test 1.

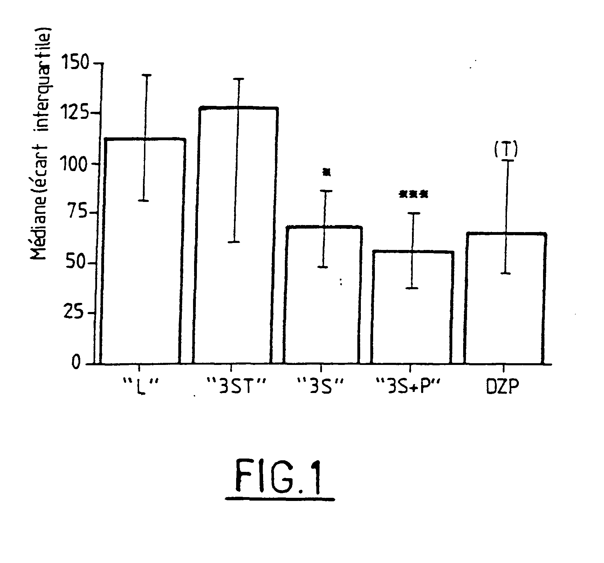

Les résultats de ce test sont illustrés par la figure 1.
Légende de la figure 1
L : lait stérile témoin
3ST : Lait fermenté par le ferment F042 puis thermisé
3S : Lait fermenté par le ferment F042
3S+P : Lait fermenté par le ferment F042 + Passiflore
DZP : Diazépam
Test de Mann-Whitney : * p<0,05 vs. L, *** p<0,005 vs. L et (T) tendance vs. L (p<0,07).

Au cours du test 1, les produits 3S et 3S+P diminuent significativement le score global de stress par rapport au produit témoin L.

Le produit 3ST ne diffère pas significativement du produit témoin L. Le diazépam tend à diminuer le score global de stress par rapport au témoin L.

Au cours du test 1, hormis le diazépam, aucun produit n'a montré d'effet sédatif.

### Score global de stress au cours du test 2.

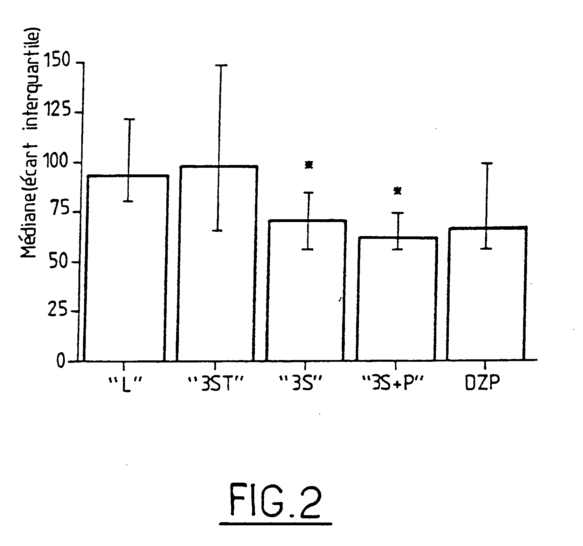

Les résultats de ce test sont illustrés par la figure 2.
Légende de la figure 2
L : lait stérile témoin
3ST : Lait fermenté par le ferment F042 puis thermisé
3S : Lait fermenté par le ferment F042
3S+P : Lait fermenté par le ferment F042 + Passiflore
DZP : Diazépam
Test de Mann-Whitney : p>0,05 *vs.* L

Au cours de ce test les produits 3S et 3S+P diminuent significativement le score global de stress par rapport au produit témoin L.

Les effets du produit 3ST et du diazépam ne diffèrent pas de manière significative du produit L.

### Conclusions

Au cours des tests 1 et 2, les produits 3S et 3S+P diminuent significativement le score global de stress par rapport au produit témoin L. Le produit 3 ST ne montre aucune activité contre le stress au cours des deux tests comparativement au produit témoin.

Le diazépam, administré à la dose de 3mg/kg p.o., pendant 6 jours avant le test 1, n'a montré qu'une tendance à réduire le score global de stress par rapport au témoin. Au cours du test 2 il ne montre pas d'effet statistiquement différent de celui du témoin. Il s'agit là d'un phénomène d'habituation propre aux produits de la famille des benzodiazépines.

On peut conclure que les produits 3S et 3S+P ont une activité anti-stress significative alors que l'effet du produit 3ST ne diffère pas de celui du témoin. Le phénomène de tolérance observé avec le diazépam, n'est pas observé avec les produits 3S et 3S+P. Aucun problème de sédation n'a été révélé avec ces deux produits.

### EXEMPLE 3 : EFFET ANXIOLYTIQUE DE LAITS FERMENTES PAR DIFFERENTES BACTERIES LACTIQUES.

Les produits testés sont les suivants :
SC008 1.1 : lait fermenté par *S. thermophilus* S242 ;
SC008 1.2 : lait fermenté par *S. thermophilus* S147 ;
3S : lait fermenté par le ferment F042 (S242+S147+S003) ;
SC008 1.5 : Sérum filtré du lait fermenté par le ferment F042 ;
L : Lait stérile témoin (95°C 10 min) ;
SC008 1.8 : lait fermenté par *L. gasseri* L012 ;
SC008 1.10 : lait fermenté par *L. acidophilus* L030 ;
SC008 1.12 : lait fermenté par *S*. *thermophilus* S003.

Les tests ont été effectués selon le protocole décrit dans l'exemple 2 ci-dessus, sur cent cinquante huit mâles Wistar/AF EOPS (Centre d'élevage IFFA-CREDO, 69 - St Germain sur Arbresles, France) d'un poids de 260 à 280 g. Après une familiarisation d'une semaine aux conditions de laboratoire, les rats ont été répartis au hasard en 13 groupes de traitement. Les rats des différents groupes ont été manipulés de la même façon et dans les mêmes conditions.

### Administration des produits.

Tous les produits ont été administrés pendant 6 jours par voie orale quotidiennement aux mêmes heures aux doses de 5 ml/kg au moyen de seringues munies de sondes de gavage intragastrique (HARVARD APPARATUS, 91 - Les Ulis, France). Le jour du test ils ont été administrés 6 heures avant le test d'enfouissement.

Le diazépam (1mg/kg, p.o., aux jours 1 et 2 ; 2 mg/kg p.o., aux jours 3 et 4 et 3 mg/kg, p.o., aux jours 5 et 6) a été mis en suspension dans un volume de 5 ml de solution aqueuse de méthylcellulose à 1%. Le jour du test, il a été administré 1 heure avant le test d'enfouissement.

Afin d'éviter les interférences, tous les rats de la même cage ont reçu le même produit.

### Variables

En plus des variables déjà décrites dans l'exemple 2, les variables suivantes de l'effet sédatif sont étudiées :
- nombre de redressements : le rat adopte la position verticale en prenant appui sur ses pattes postérieures ;
- nombre de transitions d'un coté à l'autre de la cage.

### Résultats

### Score global de stress

Comparativement au produit L, les produits SC008 1.1, 3S, SC008 1.3, SC008 1.10, SC008 1.12 et le diazépam induisent des scores globaux de stress significativement inférieurs.

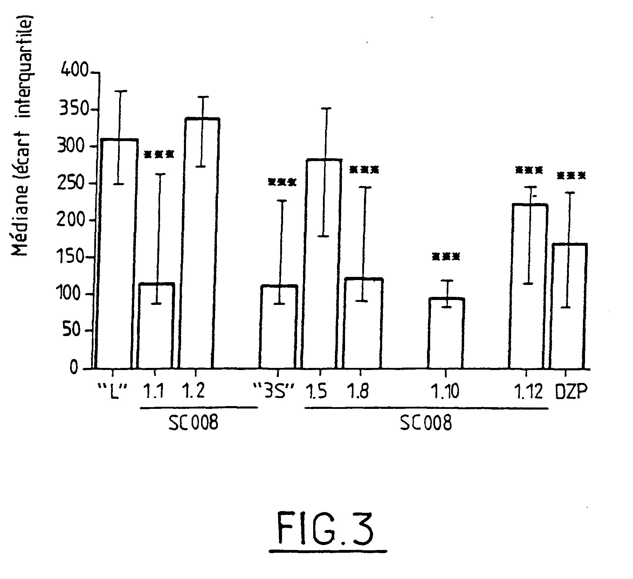

Les scores de stress induits par les produits SC008 1.2 et SC008 1.5 ne diffèrent pas significativement du produit témoin. Les résultats sont illustrés par la figure 3.
Légende de la figure 3 :
L : Lait stérile témoin (95°C 10 min.) ;
SC008 1.1 : lait fermenté par *S*. *thermophilus* S242 ;
SC008 1.2 : lait fermenté par *S. thermophilus* S147 ;
3S : lait fermenté par le ferment F042 (S242+S147+S003) ;
SC008 1.5 : Sérum filtré du lait fermenté par le ferment F042 ;
SC008 1.8 : lait fermenté par *L. gasseri* L012 ;
SC008 1.10 : lait fermenté par *L. acidophilus* L030 ;
SC008 1.12 : lait fermenté par *S. thermophilus* 5003 ;
DZP : Diazépam.
Test de Mann-Whitney :
*** p <0,005 *vs.* L.

### Effet sédatif des produits 3S et SC008 1.10

L'effet sédatif des produits 3S et SC008 1.10 par rapport au produit témoin L est évalué sur la base de l'activité locomotrice et exploratoire.

### Nombre de redressements au cours du test.

On observe une hétérogénéité dans les nombres de redressements obtenus avec les produits 3S, SC008 1.10 et L (Test de Kruskal-Wallis : H _{(d.d.l. 2)} = 6,170 ; p < 0,05). Le produit 3S entraîne une augmentation significative du nombre de redressements par rapport au produit témoin L (Test de Mann-Whitney : U = 65 ; p < 0,05). Le produit SC008 1.10 tend à augmenter le nombre de redressements par rapport au produit témoin L (Test de Mann-Whitney : U = 73 ; p < 0,07).

### Nombre de transitions d'un côté à l'autre de la cage

On n'observe pas de différence significative du nombre de transitions d'un côté à l'autre de la cage dans le cas des produits 3S, SC008 1.10 et dans celui du produit témoin L (Test de Krusdal-Mallis : H _{(d.d.l. 2)} = 0,983 : N.S.).

### Conclusion

Dans les conditions expérimentales, sur la base du score global de stress, SC008 1.1, 3S, SC008 1.8, SC008 1.10, SC008 1.12, et le diazépam montrent une activité anti-stress significative par rapport au produit témoin L. Les produits SC008 1.2 et SC008 1.5 ne présentent pas d'activité anti-stress significative par rapport au produit témoin L.

Sur la base de l'activité locomotrice et exploratoire et par rapport au témoin L, aucun effet sédatif n'a été observé avec les laits fermentés montrant une activité anti-stress.

### EXEMPLE 4 : DETERMINATION DE LA DUREE OPTIMALE D'ADMINISTRATION PAR VOIE ORALE DES PRODUITS.

Les produits testés sont les suivants : 3S : lait fermenté par le ferment F042 (S242+S147+S003) ; L : Lait stérile témoin (95°C 10 min.).

Les tests ont été effectués selon le protocole décrit dans l'exemple 2 ci-dessus, sur soixante douze rats mâles Wistar/AF EOPS (Centre d'élevage Iffa-Credo, 69 - St Germain sur Arbresles, France) d'un poids de 260 à 280 g. Après une familiarisation d'une semaine aux conditions de laboratoire, les rats ont été répartis au hasard en 6 groupes de traitement. Les rats des différents groupes ont été manipulés de la même façon et dans les mêmes conditions.

### Administration des produits

Les produits ont été administrés quotidiennement par voie orale pendant 1, 3 et 6 jours aux doses de 5 ml/kg au moyen de seringues munies de sondes de gavage intragastrique (HARVARD APPARATUS, 91 - Les Ulis, France). Le jour du test ils ont été administrés 6 heures avant le test d'enfouissement.

Les quatre rats de chaque cage ont tous reçus le même produit.

### Résultats

### Score global de stress

Administré en dose unique ou quotidiennement pendant 3 jours avant le test, le produit 3S induit un score global de stress statistiquement équivalent à celui du produit témoin L.

Administré quotidiennement pendant 6 jours avant le test, le produit 3S induit un score global de stress significativement inférieur à celui obtenu avec le produit L.

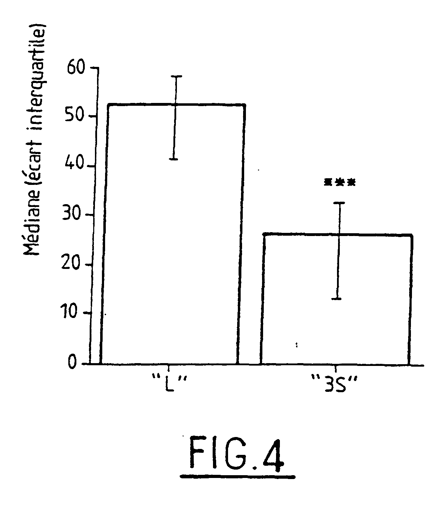

Les résultats sont illustrés par la figure 4
Légende de la figure 4 :
L : Lait stérile témoin (95°C 10 min.)
3S : lait fermenté par le ferment F042 (S242+S147+S003)
Test de Mann-Whitney :
*** p <0,005 *vs.* L.

### Conclusion

Dans les conditions expérimentales décrites ci-dessus, une administration quotidienne du produit 3S pendant 6 jours est nécessaire pour montrer une activité anti-stress significative par rapport au produit témoin L.

### EXEMPLE 5 : DETERMINATION DE L'HEURE OPTIMALE D'ADMINISTRATION PAR VOIE ORALE DES PRODUITS.

Les produits testés sont les suivants :
3S : lait fermenté par le ferment F042 (S242+S147+S003)
L : Lait stérile témoin (95°C 10 min.)

Les tests ont été effectués selon le protocole décrit dans l'exemple 2 ci-dessus, sur cent huit mâles Wistar/AF EOPS (Centre d'élevage Iffa-Credo, 69 - St Germain sur Arbresles, France) d'un poids de 260 à 280 g. Après une familiarisation d'une semaine aux conditions de laboratoire, les rats ont été répartis au hasard en 9 groupes de traitement. Les rats des différents groupes ont été manipulés de la même façon et dans les mêmes conditions.

### Administration des produits.

Préalablement au test, les produits ont été administrés par voie orale pendant 6 jours quotidiennement aux doses de 5 ml/kg au moyen de seringues munies de sondes de gavage intragastrique (Harvard Apparatus, 91 - Les Ulis, France). A l'issue de cette période, le test d'enfouissement est effectué 1, 3 ou 6 ou 24 heures après la dernière prise.

Le diazépam (1 mg/kg, p.o., aux jours 1 et 2 ; 2 mg/kg p.o., aux jours 3 et 4 et 3 mg/kg, p.o., aux jours 5 et 6) est administré dans un volume de 5 ml de solution aqueuse de méthylcellulose à 1%. La dernière administration est faite 1 heure avant le test d'enfouissement. Les quatre rats de chaque case ont tous reçu le même produit.

### Résultats

### Score global de stress

Lorsque l'administration est effectuée une heure avant le test, le produit 3S induit un score global de stress équivalent à celui obtenu avec le produit témoin L. Le score global de stress du diazépam est significativement inférieur à celui du produit témoin L.

Lorsque l'administration est effectuée 3 heures avant le test, le produit 3S induit un score global de stress qui ne diffère pas statistiquement de celui du produit témoin L.

Lorsque l'administration est effectuée 6 ou 24 heures avant le test, le produit 3S montre un score global de stress significativement inférieur à celui obtenu avec le produit témoin L.

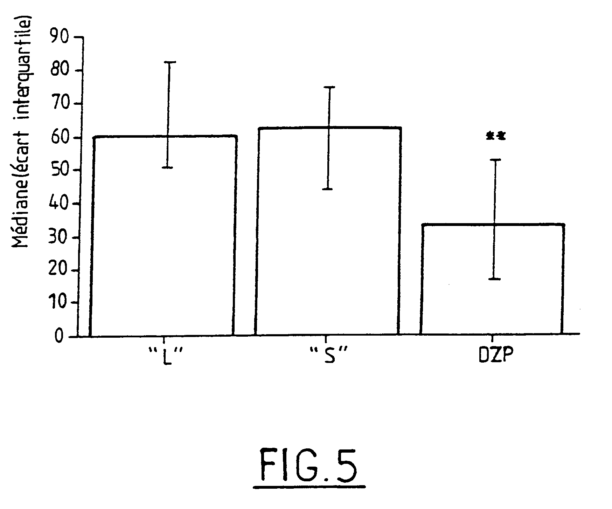

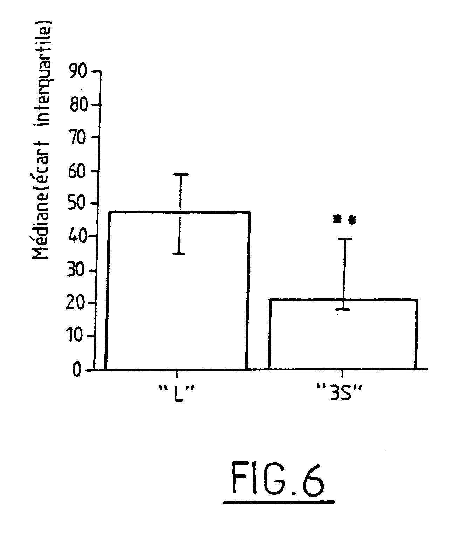

Les résultats à 1 heure et 6 heures sont illustrés par les figures 5 et 6.
Légendes des figures 5 et 6:
L : Lait stérile témoin (95°C 10 min.)
3S : Lait fermenté par le ferment F042 (S242+S147+S003)
DZP : Diazépam
Test de Mann-Whitney :
** p <0,01 vs. L.

### Conclusion

Dans les conditions expérimentales décrites ci-dessus et sur la base du score global de stress, l'activité anti-stress se manifeste entre 6 et 24 heures après l'administration du produit 3S.

### EXEMPLE 6 : RECHERCHE DU DECAPEPTIDE ISSU DE L'HYDROLYSE TRYPSIQUE DE LA CASEINE αs1 DANS UN PRODUIT FERMENTE A L'AIDE DU MELANGE 3S

La présence du décapeptide décrit dans la Demande EP 95402697 a été recherché dans le produit 3S. L'analyse a été faite par HPLC - phase inverse sur C18 (la séparation des peptides est basée sur leur hydrophobicité).
L'analyse a été effectuée sur une « précolonne » NUCLEOSIL C18-10µ - 100 mm × 4.6 mm, suivie d'une colonne analytique NUCLÉOSIL C18-3µ - 150 mm × 4,6 mm, en utilisant le gradient suivant :
A : TFA 0,1% dans H₂O
B : 90% CH3CN + 10% TFA 0,1% dans H₂O

L'élution est effectuée comme indiqué dans le Tableau III ci-après.

**Tableau III**

| | | |
|---|---|---|
| temps | A | B |
| 0 min | 90 | 10 |
| 10 min | 60 | 40 |
| 40 min | 60 | 40 |
| 45 min | 90 | 10 |
| 60 min | 90 | 10 |

La détection est effectuée à 215 nm

Dans ces conditions, le temps de rétention du décapeptide déposé sur la colonne est de 29 min.

### Préparation de l'échantillon à tester :

Produit 3S ÷ 20% CH₃CN ; agitation puis centrifugation 20 min 10 000 trs/mn.

Récupération de la phase soluble puis filtration sur filtre GELMAN 0,45µ GHP-GF (low protein binding) avant injection de 100 µl du filtrat sur la colonne de HPLC.

Aucun pic correspondant au temps de rétention du décapeptide recherché n'est retrouvé dans le filtrat du produit 3S.

## Revendications

1. Utilisation d'au moins une souche de bactérie lactique choisie parmi :
- la souche de *S. thermophilus* S242, déposée auprès de la CNCM le 24 février 1999, sous le numéro I-2130 ;
- la souche de S. *thermophilus* S003 déposée auprès de la CNCM le 24 février 1999, sous le numéro I-2129 ;
- la souche de *L. gasseri* L012 déposée auprès de la CNCM le 24 février 1999, sous le numéro I-2131 ;
- la souche de *L. acidophilus* L030 déposée auprès de la CNCM le 24 février 1999, sous le numéro I-2132,
pour l'obtention d'un anxiolytique non-sédatif.

2. Souche de bactérie lactique utilisable pour l'obtention d'un anxiolytique non-sédatif, choisie parmi :
- la souche de *S. thermophilus* S242, déposée auprès de la CNCM le 24 février 1999, sous le numéro I-2130 ;
- la souche de *S*. *thermophilus* S003 déposée auprès de la CNCM le 24 février 1999, sous le numéro I-2129 ;
- la souche de *L. gasseri* L012 déposée auprès de la CNCM le 24 février 1999, sous le numéro I-2131 ;
- la souche de *L. acidophilus* L030 déposée auprès de la CNCM le 24 février 1999, sous le numéro I-2132,

3. Utilisation selon la revendication 1, **caractérisée en ce qu'**au moins une souche de *Streptococcus thermophilus* choisie parmi la souche S242 et la souche S003, est associée avec la souche de S. *thermophilus* S147 déposée auprès de la CNCM le 30 décembre 1994, sous le numéro I-1520.

4. Ferment lactique comprenant au moins une souche de *S. thermophilus* choisie parmi la souche S242, déposée auprès de la CNCM le 24 février 1999, sous le numéro I-2130, et la souche S003 déposée auprès de la CNCM le 24 février 1999, sous le numéro I-2129.

5. Ferment lactique selon la revendication 4, comprenant en outre la souche de *S. thermophilus* S147 déposée auprès de la CNCM le 30 décembre 1994, sous le numéro 1-1520.

6. Ferment lactique selon une quelconque des revendications 4 ou 5, **caractérisé en ce qu'**il comprend en outre au moins une souche de bactérie lactique choisie dans le groupe constitué par :
- la souche de *L. gasseri* L012 déposée auprès de la CNCM le 24/02/99, sous le numéro I-2131 ;
- la souche de *L. acidophilus* L030 déposée auprès de la CNCM le 24/02/99, sous le numéro I-2132.

7. Produit ou complément alimentaire comprenant un anxiolytique non-sédatif, ledit anxiolytique non sédatif étant **caractérisé en ce qu'**il contient au moins une souche de bactérie lactique selon la revendication 2 ou un ferment lactique selon une quelconque des revendications 4 à 6, et **en ce qu'**il est constitué par du lait ou un milieu à base de lait, fermenté par ladite souche ou ledit ferment.

## Claims

1. Use of at least one strain of lactic acid bacterium selected from:
- the *S. thermophilus* S242 strain deposited in the CNCM on 24 February 1999 under the number I-2130;
- the *S. thermophilus* S003 strain deposited in the CNCM on 24 February 1999 under the number I-2129;
- the *L. gasseri* L012 strain deposited in the CNCM on 24 February 1999 under the number I-2131; and
- the *L. acidophilus* L030 strain deposited in the CNCM on 24 February 1999 under the number I-2132,
for obtaining a non-sedative anxiolytic.

2. Strain of lactic acid bacterium which can be used for obtaining a non-sedative anxiolytic, selected from:
- the *S. thermophilus* S242 strain deposited in the CNCM on 24 February 1999 under the number I-2130;
- the *S. thermophilus* S003 strain deposited in the CNCM on 24 February 1999 under the number I-2129;
- the *L. gasseri* L012 strain deposited in the CNCM on 24 February 1999 under the number I-2131; and
- the *L. acidophilus* L030 strain deposited in the CNCM on 24 February 1999 under the number I-2132.

3. Use according to Claim 1, **characterized in that** at least one strain of *Streptococcus thermophilus* selected from the S242 strain and the S003 strain is associated with the *S. thermophilus* S 147 strain deposited in the CNCM on 30 December 1994 under the number I-1520.

4. Lactic ferment comprising at least one strain of *S*. *thermophilus* selected from the S242 strain deposited in the CNCM on 24 February 1999 under the number I-2130, and the S003 strain deposited in the CNCM on 24 February 1999 under the number I-2129.

5. Lactic ferment according to Claim 4, also comprising the *S. thermophilus* S147 strain deposited in the CNCM on 30 December 1994 under the number I-1520.

6. Lactic ferment according to Claim 4 or 5, **characterized in that** it also comprises at least one strain of lactic acid bacterium selected from the group comprising:
- the *L. gasseri* L012 strain deposited in the CNCM on 24/02/99 under the number I-2131; and
- the *L. acidophilus* L030 strain deposited in the CNCM on 24/02/99 under the number I-2132.

7. Food product or complement comprising a non-sedative anxiolytic, said non-sedative anxiolytic being **characterized in that** it contains at least one strain of lactic acid bacterium according to Claim 2 or a lactic ferment according to any one of Claims 4 to 6, and **in that** it consists of milk or a milk-based medium fermented by said strain or said ferment.

## Patentansprüche

1. Verwendung wenigstens eines Milchsäurebakterienstamms ausgewählt aus:
- dem Stamm *S. thermophilus* S242, hinterlegt bei der CNCM am 24. Februar 1999 unter der Nummer I-2130;
- dem Stamm *S. thermophilus* S003, hinterlegt bei der CNCM am 24. Februar 1999 unter der Nummer I-2129;
- dem Stamm *L. gasseri* L012, hinterlegt bei der CNCM am 24. Februar 1999 unter der Nummer I-2131;
- dem Stamm *L. acidophilus* L030, hinterlegt bei der CNCM am 24. Februar 1999 unter der Nummer I-2132,
zur Herstellung eines nicht sedierenden Anxiolytikums.

2. Milchsäurebakterienstamm, der zur Herstellung eines nicht sedierenden Anxiolytikums verwendet werden kann, ausgewählt aus:
- dem Stamm *S. thermophilus* S 242, hinterlegt bei der CNCM am 24. Februar 1999 unter der Nummer I-2130;
- dem Stamm *S. thermophilus* S003, hinterlegt bei der CNCM am 24. Februar 1999 unter der Nummer I-2129;
- dem Stamm *L. gasseri* L012, hinterlegt bei der CNCM am 24. Februar 1999 unter der Nummer I-2131;
- dem Stamm *L. acidophilus* L030, hinterlegt bei der CNCM am 24. Februar 1999 unter der Nummer I-2132.

3. Verwendung nach Anspruch 1, **dadurch gekennzeichnet, dass** wenigstens ein *Streptococcus thermophilus-*Stamm, ausgewählt aus dem Stamm S242 und dem Stamm S003, mit dem Stamm *S. thermophilus* S147 assoziiert ist, der am 30. Dezember 1994 unter der Nummer I-1520 bei der CNCM hinterlegt wurde.

4. Milchferment, umfassend wenigstens einen *S. thermophilus*-Stamm, ausgewählt aus dem Stamm S242, hinterlegt bei der CNCM am 24. Februar 1999 unter der Nummer I-2130, und dem Stamm S003, hinterlegt bei der CNCM am 24. Februar 1999 unter der Nummer I-2129.

5. Milchferment nach Anspruch 4, ferner umfassend den Stamm *S. thermophilus* S147, hinterlegt bei der CNCM am 30. Dezember 1994 unter der Nummer I-1520.

6. Milchferment nach einem der Ansprüche 4 oder 5, **dadurch gekennzeichnet, dass** es ferner wenigstens einen Milchsäurebakterienstamm umfasst, ausgewählt aus der Gruppe bestehend aus:
- dem Stamm *L. gasseri* L012, hinterlegt bei der CNCM am 24. 2. 1999 unter der Nummer I-2131;
- dem Stamm *L. acidophilus* L030, hinterlegt bei der CNCM am 24. 2. 1999 unter der Nummer I-2132.

7. Nahrungsmittelprodukt oder Nahrungsergänzungsmittel, umfassend ein nicht sedierendes Anxiolytikum, wobei das nicht sedierende Anxiolytikum **dadurch gekennzeichnet ist, dass** es wenigstens einen Milchsäurebakterienstamm nach Anspruch 2 oder ein Milchferment nach einem der Ansprüche 4 bis 6 umfasst, und dadurch, dass es aus Milch oder einem Medium auf Milchbasis besteht, fermentiert durch den oben genannten Stamm oder das oben genannte Ferment.
